(19) Europäisches Patentamt
European Patent Office
Office européen des brevets

(11) Veröffentlichungsnummer: **0 257 332**
**B1**

(12) # EUROPÄISCHE PATENTSCHRIFT

(45) Veröffentlichungstag der Patentschrift: **31.10.90**

(51) Int. Cl.[5]: **C07C 29/10**, C07C 31/20

(21) Anmeldenummer: **87110975.7**

(22) Anmeldetag: **29.07.87**

(54) **Kontinuierliches Verfahren zur Herstellung von 1,2-Diolen.**

(30) Priorität: **23.08.86 DE 3628674**

(43) Veröffentlichungstag der Anmeldung:
**02.03.88 Patentblatt 88/9**

(45) Bekanntmachung des Hinweises auf die Patenterteilung:
**31.10.90 Patentblatt 90/44**

(84) Benannte Vertragsstaaten:
**AT BE CH DE FR GB IT LI NL SE**

(56) Entgegenhaltungen:
**EP-A- 0 183 028**
**DE-A- 2 256 907**
**US-A- 3 576 890**

(73) Patentinhaber: **Degussa Aktiengesellschaft, Weissfrauenstrasse 9, D-6000 Frankfurt am Main 1 (DE)**

(72) Erfinder: **Siegmeier, Rainer, Dr., Anspacher Strasse 35, D-6380 Bad Homburg v.d.H.(DE)**
Erfinder: **Prescher, Günter, Dr., Liesingstrasse 2, D-6450 Hanau 9(DE)**
Erfinder: **Maurer, Helmut, In den Steinäcker 5, D-6458 Rodenbach(DE)**

**EP 0 257 332 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Die Erfindung betrifft die kontinuierliche Herstellung vicinaler Diole, also von Diolen, deren Hydroxylgruppen an benachbarten Kohlenstoffatomen stehen, durch Verseifung der entsprechenden Epoxide.

Vicinale Diole spielen eine Rolle als Komponente bei der Polyester- und Polyurethanherstellung. sowie in der kosmetischen und pharmazeutischen Industrie.

Sie werden unter anderem durch Verseifung ihrer entsprechenden Epoxide hergestellt. Dabei wird diese Verseifung sowohl durch Zugabe von Säuren (siehe z. B. den Stand der Technik der US-PS 3 576 890), wie Alkalien (siehe z. B. DE-OS 1 793 247, DE-OS 2 203 806), wie Salzen aliphatischer Mono- oder Polycarbonsäuren (DE-OS 2 256 907), sowie primären, sekundären oder tertiären Aminsalzen oder Ammoniumsalzen (EP-OS 0 025 961) katalysiert.

Gemäß dem Verfahren der US-PS 3 576 890 lassen sich niedere Alkylenoxide mit vorzugsweise 2 bis 4 C-Atomen in die entsprechenden Alkylenglykole überführen, wenn man der Reaktionskolonne ein Reaktionsgemisch zuführt, das außer dem Epoxid Komponenten enthält, welche zwischen dem Epoxid und Wasser sieden und eine Hydrolysebarriere für nicht umgesetztes Epoxid bilden. Der Zwischensieder enthält zwingend einen niederen Ester, der mit seinen Hydrolyseprodukten, wovon die Säure auch die Hydrolyse des Epoxids katalysiert, im Gleichgewicht steht. Dieses Verfahren ist nur für die Hydrolyse von unter 100°C siedenden Epoxiden anwendbar; ferner ist die Umsetzung unvollständig, und ein nicht unbeträchtlicher Teil des Epoxids verläßt durch die sog. Barrierezone die Hydrolysezone.

Die EP-A 0 183 028 lehrt ein kontinuierliches Verfahren zur Herstellung von vicinalen Diolen durch säurekatalysierte Hydrolyse der entsprechenden Epoxide. Die Epoxide werden der Reaktionszone in Form einer Lösung in einem mit Wasser nicht mischbaren Lösungsmittel zugeführt. Dieses Verfahren erfordert die destillative Abtrennung des Lösungsmittels als Azeotrop mit Wasser und nach der Phasentrennung die Rückführung des Wassers in die Hydrolysekolonne. Das Verfahren ist nur für die Hydrolyse von $C_3$– bis $C_8$-Epoxiden geeignet.

Die Verseifung von $C_4$– bis $C_{30}$-Epoxiden kann in rein wäßrigem Medium oder in Gegenwart von Löslichkeitsvermittlern, auch als Lösungsvermittler bezeichnet, wie wasserlöslichen Ketonen oder cyclischen Äthern (DE-A 2 256 907), vorgenommen werden. Gerade in der letztgenannten Patentschrift wird ausgeführt, welche Schwierigkeiten bei einer rein wäßrigen Hydrolyse von Epoxiden ohne Anwesenheit von Löslichkeitsvermittlern bestanden, wenn man nicht Äthylenoxid, sondern höhermolekulare, wasserunlösliche Epoxide einsetzte. So waren lange Reaktionszeiten, mäßige Ausbeuten und schlechte Selektivität die Folge. Die Hydrolyse höhermolekularer Epoxide nach dem Verfahren der DE-A 2 256 907 erfordert die Verwendung wäßriger Salzlösungen, hohe Reaktionstemperaturen und zieht eine aufwendige Aufarbeitung des Reaktionsgemisches nach sich.

Die Schwierigkeiten waren naturgemäß bei der Verseifung von Epoxiden oder Epoxidschnitten, die 8 bis 30 Kohlenstoffatome enthielten, besonders groß aufgrund von deren schlechter Wasserlöslichkeit. Daher wurde nach der EP-PS 0 025 961 die diskontinuierliche Verseifung langkettiger Epoxide zu den entsprechenden Diolen unter Anwendung spezieller Katalysatoren, nämlich von Ammoniumsalzen organischer oder anorganischer Säuren, durchgeführt. Die Menge an Katalysatoren, bezogen auf das eingesetzte Epoxid, war hoch; trotz Arbeitens in einem Autoklaven lagen die Reaktionszeiten in der Regel bei 4 bis 6 Stunden, und die Ausbeuten waren nur mäßig.

Aufgabe der Erfindung ist nun ein Verfahren zur kontinuierlichen Verseifung derartiger höhermolekularer Epoxide, in dem ein rein wäßriges System bei üblichen Temperaturen und geringen Drucken eingesetzt und in Gegenwart eines sauren Katalysator gearbeitet wird.

Es wurde nun gefunden, daß sich diese Aufgabe bei der Verseifung von aliphatischen geradkettigen oder verzweigten Epoxiden mit 8–30 Kohlenstoffatomen bzw. höhermolekularen cycloaliphatischen Epoxiden sowie den entsprechenden Epoxidschnitten mit Wasser bei Drucken von 1 – 5 bar in Gegenwart eines sauren Katalysators und eines mit Wasser mischbaren organischen Lösungsmittels als Löslichkeitsvermittler lösen läßt, wenn man sowohl das Wasser und den sauren Katalysator als auch das zu verseifende Epoxid am Kopf einer Kolonne einführt und durch das in der Kolonne befindliche Dampf-Flüssigkeitsgemisch des Löslichkeitsvermittlers strömen läßt, der inert gegenüber der wäßrig sauren Phase, dem jeweiligen Epoxid und dem gebildeten Diol ist und dessen Siedepunkt bzw. Siedebereich nicht oberhalb dem des Wassers beim herrschenden Kolonnendruck liegt, wobei eine derartige Temperatur im unteren Kolonnenteil oberhalb des Kolonnensumpfes eingestellt wird, daß der aus dem Kopfteil der Kolonne herabströmende kondensierte Löslichkeitsvermittler vor Erreichen des Sumpfes wieder verdampft und am Kopf der Kolonne wieder kondensiert und die Verseifung des Epoxids zu dem entsprechenden Diol im wesentlichen in der Gas-Flüssigkeitsphase des Löslichkeitsvermittlers abläuft, worauf die gebildeten Diole und die wäßrig saure Phase aus dem Sumpf der Kolonne entnommen und in üblicher Weise aufgearbeitet werden.

Bevorzugt bildet der Löslichkeitsvermittler mit Wasser ein oder mehrere Minimumazeotrope, d. h. dessen Siedepunkt bzw. deren Siedepunkte im Druckbereich von 1 – 5 bar unterhalb des Siedepunktes von Wasser liegen. Als Löslichkeitsvermittler haben sich vor allem Diäther von Diäthylenglykolen in dem erfindungsgemäßen Verfahren bewährt, wie z. B. Dioxan, das mit Wasser ein Azeotrop bei 88°C bei 1 bar bildet.

Sehr gut eignet sich auch Diäthylenglykoldimethyläther mit einem azeotropen Siedepunkt von 99 °C bei 1 bar und tert.-Butanol mit einem solchen von 80 °C bei 1 bar.

Die anzuwendende Menge des Löslichkeitsvermittlers richtet sich nach Art und Größe der Kolonne und muß durch Handversuche festgelegt werden.

Das erfindungsgemäße Verfahren läßt sich in einer herkömmlichen Destillationskolonne durchführen, die während der Verseifung mit totalem Rücklauf betrieben wird.

Als Kolonneneinbauten kommen Füllkörper oder auch Böden, z.B. Glockenböden, in Betracht.
Der Sumpf der Kolonne kann prinzipiell durch übliche Verdampfer beheizt werden; bevorzugt ist ein Umlaufverdampfer.

Die Beheizung des Sumpfes der Kolonne wird so eingestellt, daß der kondensierte Löslichkeitsvermittler, der in den unteren Kolonnenteil zurückfließt, vor Erreichen des Sumpfes praktisch in seiner Gesamtheit wieder verdampft wird.

Dazu dient eine Temperaturmeßstelle, die die Heizung des Sumpfes derartig regelt, daß der Löslichkeitsvermittler vor Erreichen des Sumpfes - wie gesagt - praktisch vollständig wieder verdampft wird. Ort und Solltemperatur der Meßstellemüssen so gewählt werden, daß auch bei kurzen Schwankungen des Temperaturprofils praktisch kein Löslichkeitsvermittler im Sumpf mehr nachweisbar ist. Die richtige Stelle zur Temperaturmessung muß ebenfalls durch einen Handversuch vor Betrieb der Kolonne festgelegt werden. Die Regelung der Sumpftemperatur erfolgt derartig, daß bei Unterschreiten einer in den Handversuchen festgestellten Solltemperatur die Beheizung des Sumpfes so lange verstärkt wird, bis der untere Grenzwert der Temperatur zum mindesten erreicht, bevorzugt jedoch überschritten ist.

Der Kolonnensumpf besteht nur aus der wäßrig sauren Phase und dem gebildeten Diol. Durch gaschromatographische Untersuchung ist sichergestellt worden, daß er praktisch frei von dem Löslichkeitsvermittler ist. Das gebildete Diol wird durch Phasentrennung von der wäßrigen Phase getrennt. Es besitzt dann in der Regel einen Reinheitsgrad, der für seine Weiterverwendung ausreicht. Nur bei speziellen Anforderungen kann es destillativ gereinigt werden.

Als Epoxide mit 8 - 30 Kohlënstoffatomen eignen sich Epoxide der Formel

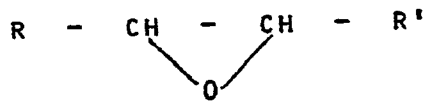

in der R einen Alkylrest und R' einen Alkylrest oder Wasserstoff darstellen.
R und R' können Alkylreste mit zusammen bis zu 28 Kohlenstoffatomen sein, R und R' sind zusammen gleich oder größer als 6.

Als saure Katalysatoren eignen sich Mineralsäuren und starke organische Säuren, z.B. Schwefelsäure, Perchlorsäure, Methansulfon- oder Toluolsulfonsäure. Bevorzugt sind Schwefel- oder Perchlorsäure.

Die Konzentrationen der wäßrigen Säuren liegen zwischen 0,1 und 5 Gewichtsprozent, bevorzugt zwischen 0,5 und 2 Gewichtsprozent.
Das Verhältnis von Epoxid zu Wasser liegt im allgemeinen bei 1 : 2 bis 1 : 10, bevorzugt bei 1 : 5 bis 1 : 10.
Der saure Katalysator wird bevorzugt zusammen mit dem Wasser in den Kopfteil der Kolonne gegeben, obwohl eine getrennte Zugabe von beiden möglich ist.

Bei Inbetriebnahme der Kolonne wird im Sumpf ein Gemisch aus Wasser und Löslichkeitsvermittler vorgelegt, in dem Wasser in solcher Menge vorliegt, daß der Löslichkeitsvermittler als Azeotrop vollständig aus dem Sumpf verdampft, gleichzeitig aber die Sumpfheizung betrieben werden kann. Dieses wird zum Sieden erhitzt und nach Erreichen eines stationären Dampf-Flüssigkeitsgleichgewichts der Sumpf auf Freiheit vom Löslichkeitsvermittler durch Gaschromatographie überprüft.

Anschließend wird das Epoxid über Leitung 11 am Kopf der Kolonne 20 eingeführt (siehe Abbildung 1). Die wäßrige Phase, die aus Frischwasser und bevorzugt noch zusätzlich aus dem bei der Abtrennung des Diols anfallenden sauren Rückwasser besteht, tritt ebenfalls am Kopf der Kolonne 20 ein, und zwar über Leitung 10. Die wäßrige Phase enthält in jedem Fall, d.h. mit oder ohne Rückwasser, bevorzugt den sauren Katalysator. Epoxid wie Wasser und saurer Katalysator durchströmen gemeinsam die Dampf-Flüssigkeitsphase des Löslichkeitsvermittlers innerhalb der Kolonne 20. Aus dem Sumpf der Kolonne 20 wird der Sumpfinhalt über Leitung 22 in ein Phasentrenngefäß 30 geleitet, die wäßrig saure Phase als Rückwasser über Leitung 31 zurück in Leitung 10 am Kopf der Kolonne 20 geführt und das Diol über Leitung 32 abgenommen.
21 ist die Temperaturmeßstelle.

Der technische Fortschritt des erfindungsgemäßen Verfahrens liegt einmal in der Möglichkeit, langkettige Diole kontinuierlich in hohen Ausbeuten, die um oder über 90 % liegen, zu erhalten. Dabei werden sehr niedrige Verseifungsdrucke, wie Atmosphärendruck, oder nur wenig darüber liegende Drucke angewendet, was mäßige Verseifungstemperaturen bedeutet. Hierdurch sind die anzuwendenden Apparate einfach, wie z.B. übliche Destillationskolonnen.

Das erfindungsgemäße Verfahren wird durch die weitere Möglichkeit, das zum Verseifen verwendete Wasser zu recyclieren, nicht nur umweltfreundlich, sondern es ist auch sehr wirtschaftlich, da kein Abfall entsteht.
Wesentlich ist auch,daß der Löslichkeitsvermittler als stationäre Phase in der Kolonne verbleibt und sich nicht im verseiften Gemisch wiederfindet; seine Abtrennung aus diesem Gemisch ist daher überflüssig.
Die erhaltenen Diole haben schließlich einen für ihre hauptsächlichen Verwendungsgebiete völlig ausreichenden Reinheitsgrad.

Die Erfindung wird an den in Tabelle I zusammengefaßten Beispielen näher erläutert:

In der Tabelle bedeuten:
E-Einsatz g/h= Epoxid-Einsatz g/h
Wasser g/h= Wasser-Einsatz g/h
Katalysator Menge= bezogen auf die wäßrige Phase
LV= Löslichkeitsvermittler

Der Umsatz war praktisch quantitativ bei Verseifungszeiten von 0,5 - 1 h.
Die Versuche wurden bei 1 bar durchgeführt.

Tabelle I

| Epoxid | E-Einsatz (g/h) | Wasser (g/h) | Katalysator-Menge | LV | Ausbeute (%) |
|---|---|---|---|---|---|
| 1-Octenoxid | 128,4 | 827 | $HClO_4$ 0,6 % | Dioxan | 93,0 |
| 1-Octenoxid | 127,2 | 828,7 | $H_2SO_4$ 0,6 % | Dioxan | 90,3 |
| 1-Dodecenoxid | 185 | 846,6 | $HClO_4$ 1,0 % | Dioxan | 92,2 |
| 1-Dodecenoxid | 103,5 | 906,3 | $H_2SO_4$ 1,0 % | Dioxan | 89,0 |
| 1-Dodecenoxid | 185,6 | 842,2 | $H_2SO_4$ 1,0 % | t-Butanol | 87,1 |
| 1-Octadecenoxid | 182 | 866,2 | $HClO_4$ 1,5 % | Dioxan | 91 |

## Patentansprüche

1. Kontinuierliches Verfahren zur Herstellung von 1,2-Diolen durch Verseifung von aliphatischen geradkettigen oder verzweigten Epoxiden mit 8 bis 30 Kohlenstoffatomen bzw. höhermolekularen cycloaliphatischen Epoxiden sowie deren Epoxidschnitten mit Wasser bei Drucken von 1–5 bar in Gegenwart eines sauren Katalysators und eines mit Wasser mischbaren organischen Lösungsmittels als Löslichkeitsvermittler, dadurch gekennzeichnet, daß man das Wasser und den sauren Katalysator und das zu verseifende Epoxid am Kopf einer Kolonne einführt und durch das in der Kolonne befindliche Dampf-Flüssigkeitsgemisch des Löslichkeitsvermittlers strömen läßt, der inert gegenüber der wäßrig sauren Phase, dem jeweiligen Epoxid und dem gebildeten Diol ist und dessen Siedepunkt bzw. Siedebereich nicht oberhalb dem des Wassers beim herrschenden Kolonnendruck liegt, wobei eine derartige Temperatur im unteren Kolonnenteil oberhalb des Kolonnensumpfes eingestellt wird, daß der aus dem Kopfteil der Kolonne herabströmende kondensierte Löslichkeitsvermittler vor Erreichen des Sumpfes wieder verdampft und am Kopf der Kolonne wieder kondensiert, und die Verseifung des Epoxids zu dem entsprechenden Diol im wesentlichen in der Gas-Flüssigkeitsphase des Löslichkeitsvermittlers abläuft, worauf man die gebildeten Diole und die wäßrig saure Phase aus dem Sumpf der Kolonne entnimmt und in üblicher Weise aufarbeitet.

2. Verfahren nach Anspruch 1, dadurch gekennzeichnet, daß man einen Löslichkeitsvermittler einsetzt, der mit Wasser ein oder mehrere Minimumazeotrope bildet.

3. Verfahren nach Anspruch 1 und 2, dadurch gekennzeichnet, daß man als Löslichkeitsvermittler Diäther von Diäthylenglykol, bevorzugt Diäthylenglykoldimethyläther, einsetzt.

4. Verfahren nach Anspruch 1 und 2, dadurch gekennzeichnet, daß man Dioxan als Löslichkeitsvermittler einsetzt.

5. Verfahren nach Anspruch 1 und 2, dadurch gekennzeichnet, daß man tertiäre Alkohole, bevorzugt tert.-Butanol, als Löslichkeitsvermittler einsetzt.

6. Verfahren nach Anspruch 1 bis 5, dadurch gekennzeichnet, daß man ein Verhältnis von Epoxid zu Wasser von 1:2 bis 1:10, bevorzugt 1:5 bis 1:10, einsetzt.

## Claims

1. A continuous process for the preparation of 1,2-diols by the saponification of aliphatic straight chained or branch chained epoxides having 8 to 30 carbon atoms or relatively high molecular weight cycloaliphatic epoxides and epoxide fractions thereof with water at pressures from 1 to 5 bar in the presence of an acid catalyst and a water-miscible organic solvent as solubilizing agent, characterised in that the water and acid catalyst and the epoxide to be saponified are introduced at the head of a column and caused to flow through the vapour-liquid mixture of solubilizing agent in the column, which solubilizing agent is inert towards the aqueous acid phase, the epoxide used and the diol formed and has a boiling point or boiling range not above that of water at the prevailing column pressure, the lower part of the column above the sump of the column being adjusted to such a temperature that the condensed solubilizing agent flowing down from the head of the column evaporates before reaching the sump and condenses again at the head of the column, and saponification of the epoxide to form the corresponding diol takes place substantially in the gasliquid phase of the solubilizing agent, whereupon the diols formed and the aqueous acid phase are removed from the sump of the column and worked up in the usual manner.

2. A process according to Claim 1, characterised in that the solubilizing agent used is one which forms one or more minimum azeotropic mixtures with water.

3. A process according to claims 1 and 2, characterised in that the solubilizing agent used is a diether of diethylene glycol, preferably diethylene glycol dimethylether.

4. A process according to Claims 1 and 2 characterised in that dioxane is used as solubilizing agent.

5. A process according to claims 1 and 2, characterised in that tertiary alcohols are used as solubilizing agents, preferably tertiary butanol.

6. A process according to claims 1 to 5, characterised in that the ratio of epoxide to water used is from 1:2 to 1:10, preferably from 1:5 to 1:10.

## Revendications

1. Procédé continu de préparation de diols 1,2 par saponification d'époxydes aliphatiques linéaires ou ramifiés de 8 à 30 atomes de carbone ou bien d'époxydes cycloaliphatiques de mousse moléculaire élevée ainsi que les coupes d'époxydes correspondantes par l'eau à des pressions de 1-5 bar en présence d'un catalyseur acide et d'un solvant organique miscible à l'eau en tant que dissolvant, caractérisé en ce qu'on introduit l'eau et le catalyseur acide ainsi que l'oxyde à saponifier en tête d'une colonne et on laisse s'écouler à travers le mélange vapeur-liquide de dissolvant se trouvant dans la colonne, qui est inerte vis-à-vis de la phase aqueuse acide de l'époxyde concerné et diol formé et dont le point d'ébullition ou le domaine d'ébullition ne se situe pas au-dessus de celui de l'eau à la pression régnant dans la colonne, en réglant une telle température dans la partie inférieure de colonne audessus du pied de colonne, que le dissolvant condensé s'écoulant de la tête de colonne est à nouveau vaporisé avant d'atteindre le pied et

en tête de colonne, il est à nouveau condensé et la saponification de l'époxyde en diol correspondant a lieu essentiellement dans la phase gaz-liquide du dissolvant, après quoi, on prélève les diols formés et la phase aqueuse acide en pied de colonne et on les traite de manière usuelle.

2. Procédé selon la revendication 1, caractérisé en ce qu'on utilise un dissolvant qui forme avec l'eau un ou plusieurs azéotropes positifs.

3. Procédé selon les revendications 1 et 2, caractérisé en ce qu'on utilise comme dissolvant du diéther de diéthylèneglycol de préférence de l'éther diméthylique de diéthylèneglycol.

4. Procédé selon les revendications 1 et 2, caractérisé en ce qu'on utilise comme dissolvant du dioxane.

5. Procédé selon les revendications 1 et 2, caractérisé en ce qu'on utilise comme dissolvant des alcools tertiaires, de préférence le tert-butanol.

6. Procédé selon les revendications 1 à 5, caractérisé en ce qu'on utilise une proportion d'époxyde à l'eau de 1:2 à 1:10, de préférence de 1:5 à 1:10.

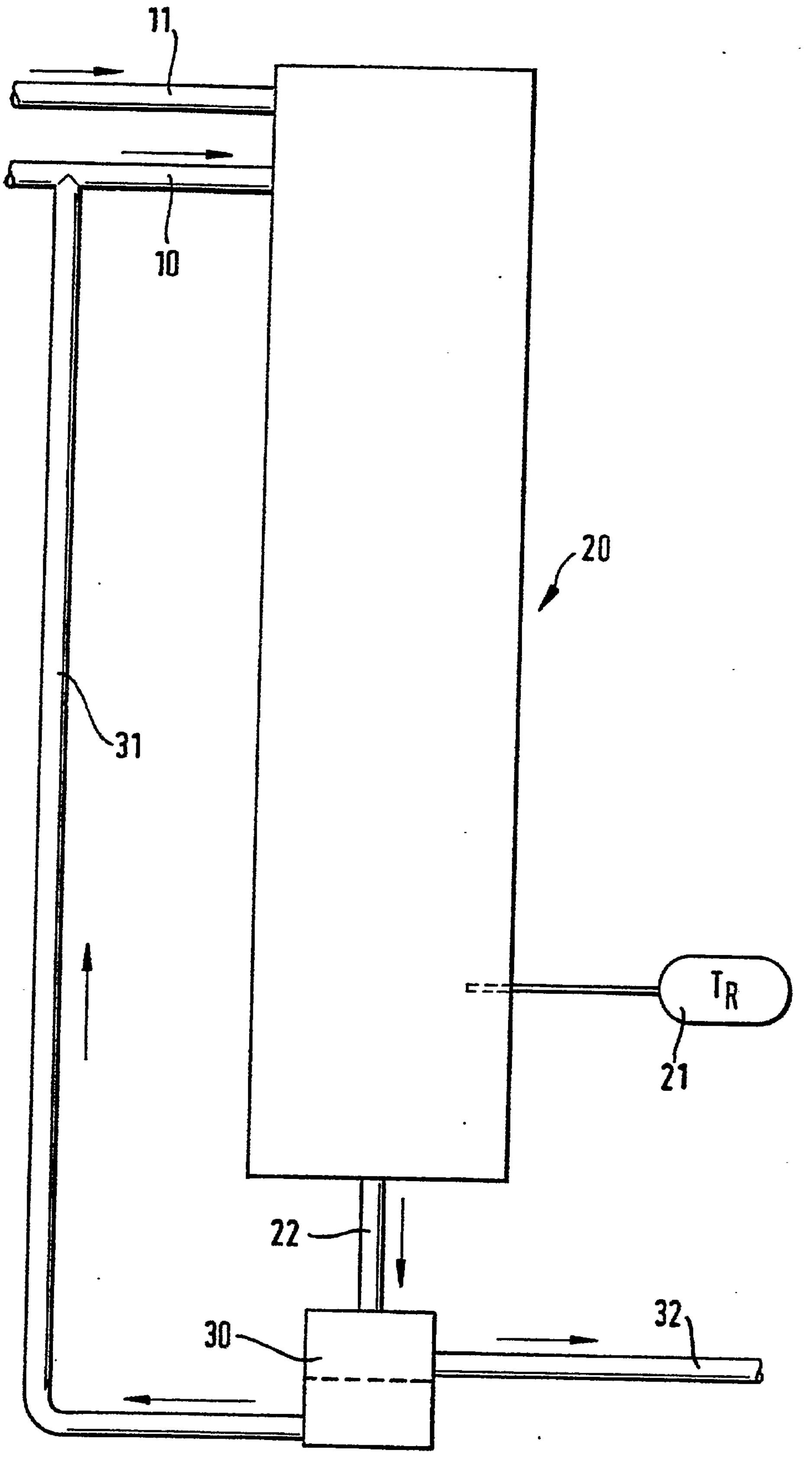
11
10
20
31
$T_R$
21
22
30
32